# EUROPEAN PATENT SPECIFICATION

(11) **EP 3 220 978 B1**
(45) Date of publication and mention of the grant of the patent: **11.08.2021**
(21) Application number: 15795167.4
(22) Date of filing: 17.11.2015
(51) Int. Cl.: A61P 3/08, A61P 43/00, A61M 5/145, A61M 5/142, A61M 5/172, A61B 5/00, A61B 5/145

(54) **APPARATUS AND METHODS FOR ADMINISTERING A PHARMACEUTICAL AGENT**
VORRICHTUNG UND VERFAHREN ZUR VERABREICHUNG EINES PHARMAZEUTISCHEN WIRKSTOFFS
APPAREIL ET PROCÉDÉS POUR ADMINISTRER UN AGENT PHARMACEUTIQUE

(30) Priority: 17.11.2014 EP 14193444
(43) Date of publication of application: 27.09.2017
(73) Proprietor: Zealand Pharma A/S, 2860 Søborg (DK)
(72) Inventor: STEENSBERG, Adam, 2000 Frederiksberg (DK)
(74) Representative: Mewburn Ellis LLP
(86) International application number: PCT/EP2015/076840
(87) International publication number: WO 2016/079128

(56) References cited:
- WO-A1-2010/142734
- WO-A1-2013/128912
- WO-A2-00/10628
- WO-A2-2007/049961
- WO-A2-2009/015389
- CN-A- 102 600 526
- US-A1- 2005 192 557
- US-A1- 2006 173 406
- US-A1- 2011 160 655
- US-A1- 2013 144 254
- US-A1- 2014 107 607
- US-A1- 2014 276 583
- None

## Description

The present application claims the benefit of European patent application n° EP14193444.8 filed on November 17th, 2014.

### TECHNICAL FIELD

This application relates to apparatus and methods for controlling blood glucose levels in a diabetic patient.

### BACKGROUND

Hypoglycemia is a medical emergency that involves an abnormally diminished content of glucose in the blood. It can produce a variety of symptoms and effects but the principal problems arise from an inadequate supply of glucose to the brain, resulting in impairment of function. Effects can range from mild dysphoria to more serious issues such as seizures, unconsciousness, and permanent brain damage or death.

While conscious, most diabetic patients can manage their blood glucose level by periodically monitoring their levels and by recognizing symptoms associated with a low blood glucose level. In the conscious state, upon a determination that blood glucose level is low, the patient may self-administer treatment in the form of ingesting rapid-acting carbohydrates or by self-injecting a blood glucose level elevating drug, such as glucagon. If severe hypoglycemia occurs the patients may become confused or unconscious and having a third person injecting a glucose elevating drug, such as glucagon may be needed to increase blood sugar. Hypoglycemia that occurs at night while the diabetic patient is asleep is particularly dangerous, since the patient is typically oblivious to the hypoglycemic symptoms and, as a result, is unable to self-administer treatment or seek treatment from others.
Various devices and systems for controlling and / or balancing blood sugar levels have been proposed, for example in US 2014/107607, WO 2013/128912, US 2006/173406, US 2011/160655, WO 00/10628, WO 2009/015389, US 2005/192557, WO 2010/142734, US 2013/144254, WO 2007/049961, CN 102 600 526 and US 2014/276583.

### SUMMARY OF THE DISCLOSURE

In order to provide treatment to a diabetic patient suffering from hypoglycemia while being unable to self-administer treatment for hypoglycaemia, such as glucagon injections, disclosed herein are apparatus and methods that provide for the injection of a glucose regulating product such as glucagon into the patient upon a determination that the patient's blood glucose level is below a designated threshold indicative of the onset occurrence of a hypoglycemic event.

According to some implementations an emergency injection device adapted to be worn on the body of a patient is provided with the device comprising: a housing having a sterile inner chamber, the housing having a bottom wall with a through opening that extends between an inner surface and an outer surface, a portion of the outer surface that possesses the through opening being configured to be worn in contact with the body of the patient, a septum positioned in and/or over the through hole opening that assists in maintaining the sterile integrity of the sterile chamber; and an injector located within the sterile inner chamber, the injector comprising a reservoir containing an agent (e.g. glucagon) for treating hypoglycaemia and a needle comprising a first end portion connected to the reservoir and a second end portion adapted to be inserted into the body of the patient, the second end portion of the needle being movable between a first position wherein the second end portion of the needle partially resides in the septum and a second position where the second end portion of the needle extends through the septum and at least partially resides external to the bottom wall, the septum and second end portion of the needle being constructed so that the second end portion of the needle is capable of piercing the septum when the second end portion of the needle is moved between the first position and the second position.

According to some implementations an injection device adapted to be worn on the body of a patient while the patient is unable to self-administer a pharmaceutical agent, the device comprising: an injector having a reservoir containing a first pharmaceutical agent which in certain implementations may be selected to counteract the activity of a second pharmaceutical agent administered separately to the patient, and a needle comprising a first end portion connected to the reservoir and a second end portion adapted to be inserted into the body of the patient; an acoustical alarm; and a control assembly comprising a signal receiver configured to receive signals from a sensor that is capable of monitoring a relevant biomarker in the patient, the control assembly electrically coupled to the acoustical alarm and configured to activate the acoustical alarm upon a signal received in the signal receiver being indicative of a presence of the biomarker, the control assembly configured to automatically cause the injector to administer through the needle a dose of the first pharmaceutical agent in the reservoir to the patient after a predetermined period of time of the signal receiver receiving the signal indicative of the presence of the biomarker, the control assembly having a manually activated switch that when activated prior to the expiration of the predetermined period of time causes the control assembly to abort the automatic injection of the first pharmaceutical agent. According to some implementations the first pharmaceutical agent is glucagon and the second pharmaceutical agent is an anti-diabetic agent. The biomarker in question could be directly or indirectly related to blood glucose level. According to some implementations the first pharmaceutical agent is glucagon and the second pharmaceutical agent is insulin. The biomarker in question could be directly or indirectly related blood glucose level. According to some implementations the first pharmaceutical agent is adrenalin and the second pharmaceutical agent is a therapeutical antibody. The biomarker in question could be a significant drop in blood pressure.

According to some implementations an emergency injection device adapted to be worn on the body of a patient is provided with the device comprising an injector with a reservoir containing an agent (e.g. glucagon) for treating hypoglycaemia and a needle comprising a first end portion connected to the reservoir and a second end portion adapted to be inserted into the body of the patient; an acoustical alarm; and a control assembly comprising a signal receiver configured to receive signals from a glucose sensor, the control assembly being electrically coupled to the acoustical alarm and configured to activate the acoustical alarm upon a receiving a signal in the signal receiver that is indicative of hypoglycaemia, the control assembly being configured to automatically cause the injector to administer through the needle a dose of the agent in the reservoir to the patient after a predetermined period of time from the signal receiver receiving the signal indicative of hypoglycaemia, the control assembly having a manually activated switch that when activated prior to the expiration of the predetermined period of time causes the control assembly to abort the automatic injection of the agent. According to some implementations the injector resides in a sterile chamber inside a first housing, the first housing having a bottom wall with a through opening that extends between an inner surface and an outer surface, a portion of the outer surface that possesses the through opening being configured to be worn in contact with the body of the patient; a septum positioned in and/or over the through hole opening assists in maintaining the sterile integrity of the sterile chamber, the second end portion of the needle being movable between a first position where the second end portion of the needle partially resides in the septum and a second position where the second end portion of the needle extends through the septum and at least partially resides external to the bottom wall, the septum and second end portion of the needle being constructed so that the second end portion of the needle is capable of penetrating the septum when the second end portion of the needle is moved between the first position and the second position.

According to some implementations an emergency injection device adapted to be worn on the body of a patient is provided with the device configured to carry out the method of: (i) receiving a signal from a glucose sensor, (ii) processing the received signal to determine whether a low glucose level is detected, (iii) activating an alarm to alert the patient when a low glucose level is detected, (iv) after a predetermined period of time after activating the alarm, administering an emergency dose of a hypoglycemia treatment agent (e.g. glucagon) to the patient, (v) at a time T after the emergency dose has been administered to the patient, receiving a signal from the glucose sensor to determine if a low glucose level persists, and (vi) if a low glucose level persists, activating the alarm again to alert the patient. According to some implementations the emergency injection device includes a controller in the form of a computer and a non-transitory computer readable medium storing computer readable program code for causing the computer to perform a method.

According to some implementations an emergency injection device adapted to be worn on the body of a patient is provided, with the device configured to carry out the method of: (i) receiving a signal from a glucose sensor, (ii) processing the received signal to determine whether a low glucose level is detected, (iii) if a low glucose level is detected activating an alarm to alert the patient, (iv) delaying the administration of an emergency dose of a hypoglycemia treatment agent (e.g. glucagon) for a predetermined period of time after activating the alarm, (v) determining during the predetermined period of time whether the patient has manually deactivated the alarm, and (vi) upon determining that the alarm has been deactivated aborting the administration of the emergency dose. According to other implementations the device is further configured to carry out the method of activating the alarm again if the low glucose level persists for a predetermined period of time after the user deactivates the alarm. According to some implementations the emergency injection device includes a controller in the form of a computer and a non-transitory computer readable medium storing computer readable program code for causing the computer to perform a method.

According to some implementations a system is provided that includes a glucose sensor adapted to be worn on the body of a patient and that when worn is able to determine the blood glucose level of the patient. The system also includes an emergency injection device also adapted to be worn on the body of the patient, the emergency injection device comprising an injector with a reservoir containing an agent (e.g. glucagon) for treating hypoglycaemia and a needle comprising a first end portion connected to the reservoir and a second end portion adapted to be inserted into the body of the patient; an acoustical alarm; and a control assembly comprising a signal receiver configured to receive signals from the glucose sensor, the control assembly being electrically coupled to the acoustical alarm and configured to activate the acoustical alarm upon a receiving a signal in the signal receiver that is indicative of hypoglycaemia, the control assembly being configured to automatically cause the injector to administer through the needle a dose of the agent in the reservoir to the patient after a predetermined period of time from the signal receiver receiving the signal indicative of hypoglycaemia, the control assembly having a manually activated switch that when activated prior to the expiration of the predetermined period of time causes the control assembly to abort the automatic injection of the agent. According to some implementations the glucose sensor comprises a stand-alone component intended to be worn spaced-apart from the emergency injection device. According to other implementations the glucose sensor is integrated into, or otherwise structurally coupled to, the emergency injection device. According to either of the aforementioned implementations the glucose sensor may communicate with the control assembly controller via wired or wireless communications. In the latter case, the glucose sensor may include a short-range signal generator that is capable of communicating with the signal receiver of the emergency injection device.

According to some implementations the system further includes an insulin pump that is also adapted to be worn on the body of the patient. The insulin pump includes a controller that regulates the administration of insulin to the patient. According to some implementations the glucose sensor comprises a stand-alone component that may be worn spaced-apart from the emergency injection device and the insulin pump injection site. In such an implementation the glucose sensor includes a short-range signal generator that is capable of communicating with the signal receiver of the emergency injection device and also a signal receiver of the insulin pump. The signal receiver of the insulin pump may or may not be comprised in the insulin pump's controller. In any event, the controller processes the signals received from the glucose sensor in order regulate the infusion of insulin to the patient. According to other implementations, as noted above, the glucose sensor may be integrated into or otherwise structurally coupled to the emergency injection device. In such implementations the glucose sensor may communicate with the emergency injection device via a wired connection.

The emergency injection device and insulin pump controllers may comprise computing apparatus and computer programs that may be executed on the computing apparatus. The computer programs may be in the form of source code, object code, a code intermediate source and object code such as in partially compiled form, or in any other form suitable for use in the implementation of the processes according to the invention. The carrier may be any entity or device capable of carrying the program. For example, the carrier may comprise a storage medium, such as a ROM, for example a CD ROM or a semiconductor ROM, or a magnetic recording medium. Further, the carrier may be a transmissible carrier such as an electrical or optical signal which may be conveyed via electrical or optical cable or by radio or other means. When the program is embodied in a signal which may be conveyed directly by a cable or other device or means, the carrier may be constituted by such cable or other device or means. Alternatively, the carrier may be an integrated circuit in which the program is embedded, the integrated circuit being adapted for performing, or for use in the performance of, the relevant processes.

### BRIEF DESCRIPTION OF THE DRAWINGS

Figures 1A-C illustrate an automatic emergency injection device according to one implementation.
Figure 1D shows a system according to one implementation comprising an automatic emergency injection device and a glucose sensor attached to the body of a diabetic patient.
Figure 1E shows a system according to one implementation comprising an automatic emergency injection device, insulin pump and glucose sensor attached to the body of a diabetic patient.
Figure 2 illustrates an automatic emergency injection device according to another implementation.
Figure 3A illustrates an automatic emergency injection device having an integrated glucose sensor according to one implementation.
Figure 3B shows the automatic emergency injection device of Figure 3A attached to the body of a diabetic patient.
Figure 3C shows a system according to one implementation comprising an insulin pump and an automatic emergency injection device having an integrated glucose sensor attached to the body of a diabetic patient.
Figure 4 illustrates an automatic emergency injection device according to another implementation.
Figure 5 illustrates a manually activated emergency injection device according to one implementation.
Figure 6 illustrates an automatic emergency injection device having an integrated glucose sensor according to another implementation.
Figure 7 illustrates an automatic emergency injection device according to another implementation.
Figure 8 illustrates a flow chart of an automatic emergency injection device control method according to one implementation.
Figure 9A shows a distal end segment of a needle embedded in a septum according to one implementation when an injector connected to the needle resides in a ready position.
Figure 9B shows a distal end segment of a needle residing in a recess of a septum according to one implementation when an injector connected to the needle resides in a ready position.

### DETAILED DESCRIPTION

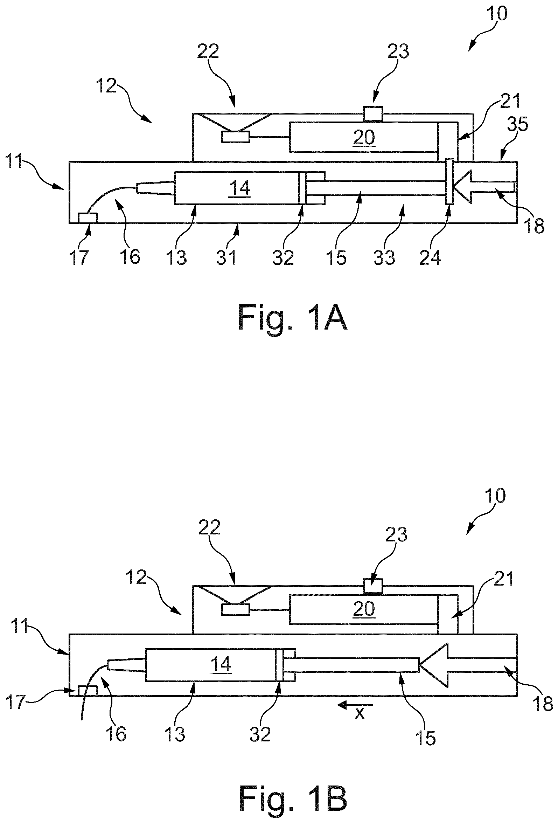

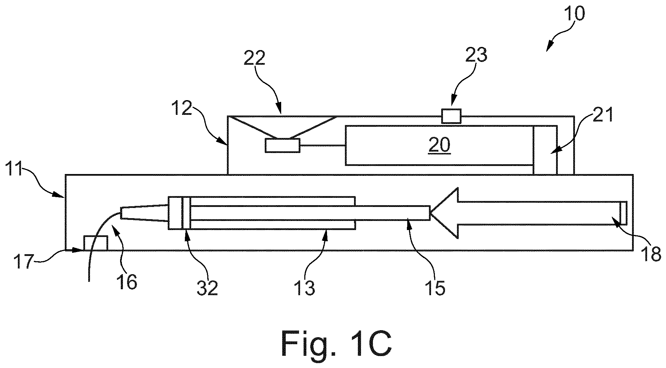

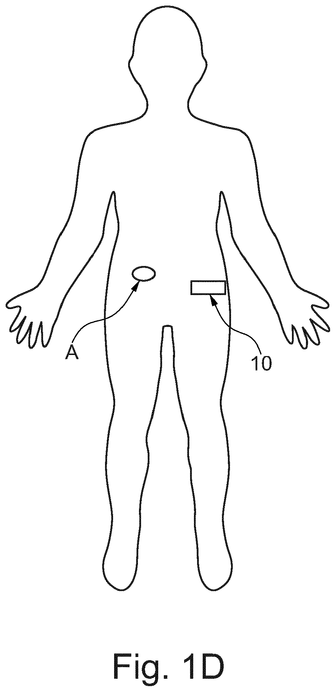

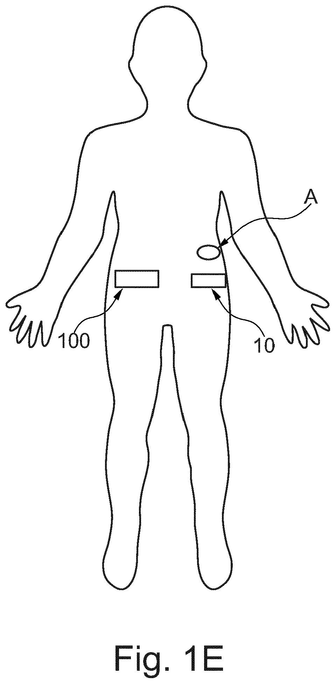

FIGS. 1A-1C illustrate an emergency injection device 10 in accordance with one implementation. The device 10 includes an injector 13 situated within a first housing 11. According to some implementations the injector 13 is able to translate a distance in the direction X from a ready position as depicted in FIG. 1A to an injection position as depicted in FIG. 1B. As will be described in more detail below, the translation of the injector 13 enables a needle 16 attached to an end of the injector 13 to be advanced through a septum 17 situated in a bottom wall 31 of the housing 11 so that after the injector 13 has assumed the injection position a distal end portion 16a of the needle 16 projects from the bottom wall 31 as shown in FIG. 1B. The injector 13 includes a reservoir 14 containing an agent for treating hypoglycemia, such as glucagon. The needle 16 possesses an internal through lumen that extends between proximal and distal ends of the needle with the proximal end of the needle 16 being in fluid communication with the reservoir 14. A plunger 32 located within the reservoir 14 is moveable from a first position as shown in FIG. 1A to a second position as shown in FIG. 1C and functions to expel the treatment agent from the reservoir 14 upon a shaft 15 coupled to the plunger 32 being acted upon by a spring element 18.

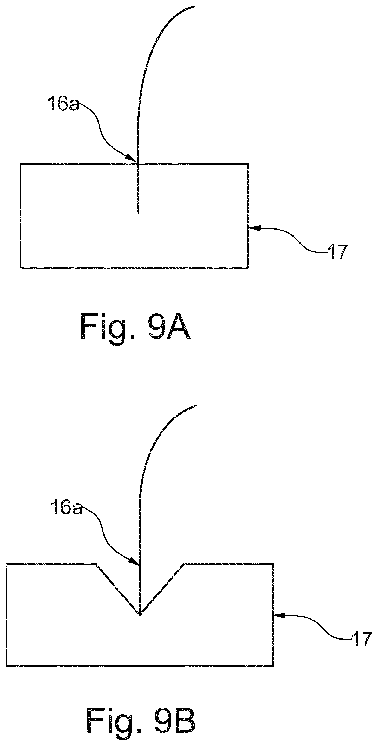

According to some implementations, the inner chamber 33 of the housing 11 is maintained in a sterile condition at least when the injector 13 is in the ready position. As noted above, a septum 17 located in the bottom wall 31 of the housing 11 provides an exit port by which the distal end portion 16a of the needle 16 may be advanced to a position outside the housing 11. The construction and material of each of the needle 16 and the septum 17 enables the distal end portion 16a of the needle 16 to be advanced through the septum 17 as the injector 13 is moved from the ready position to the injection position. According to some implementations the needle 16 is made of a flexible or semi-flexible metal or plastic material. The needle 16 may comprise any type of conduit capable of transporting the hypoglycemia treatment agent from the reservoir 14 to the injection site of the patient. According to some implementations, when the injector 13 is in the ready position at least a segment of the distal end portion 16a of the needle 16 is positioned inside the septum 17, being embedded in the septum 17 as shown in FIG. 9A or otherwise residing in a recess of the septum 17 as shown in FIG. 9B. According to some implementations at least a segment of the distal end portion 16a of the needle 16 is embedded in the septum 17 as a result of, for example, having been pierced through a portion of the septum 17. According to some implementations the segment residing in the septum 17 when the injector 13 is in the ready position is oriented perpendicular, or substantially perpendicular, to the bottom wall 31 of the housing 11 as shown in FIGS. 9a and 9B. When the needle 16 is advanced through the septum 17, such an arrangement encourages the distal end of the needle 16 to take a path in alignment with the perpendicular orientation. That is, it encourages the distal end portion 16a to assume a predominately perpendicular orientation with respect to the bottom wall 31 when the distal end portion is advanced to reside outside the housing 11.

The device 10 includes a controller 20 that has or otherwise communicates with a signal receiver capable of receiving a wireless signal from a glucose sensor that periodically or continuously monitors the glucose level of the patient. As shown in FIG. ID, the glucose sensor A is typically worn on the body of the patient and may comprise any of a variety of types of glucose sensor known in the market. According to some implementations the glucose sensor A subcutaneously monitors the glucose level of the patient and possesses a signal transmitter that transmits to the emergency injection device 10 information regarding the detected glucose level of the patient. The controller 20 is configured to process signals received from the glucose sensor A to determine if the signal is indicative of hypoglycemia. The controller 20 is electrically coupled to an alarm 22 and is configured to activate the alarm 22 when it is determined that hypoglycemia is detected in the patient. According to some implementations the alarm 22 is an acoustical alarm that produces a sound sufficient to wake the patient when the patient is asleep. According to some implementations the acoustical alarm produces sounds of greater than 50 decibels, 60 decibels, 70 decibels, 80 decibels or 90 decibels. In lieu of the acoustical alarm or in conjunction therewith, the alarm 22 may also comprise a vibrating element, electrical stimulator or other element or set of elements constructed to stimulate a patient's sense of touch.

The controller 20 also controls an electrical actuator 21 that is capable of altering the position of a stop element 24 coupled thereto. In a first position, as shown in FIG. 1A, the stop element 24 is positioned between the spring element 18 and an end of the plunger shaft 15 to prevent the spring element 18 from acting on the plunger shaft 15. When the actuator 21 is activated by the controller 20 the stop element 24 is moved away from the first position to permit the spring element 18 to act on the plunger shaft 15 as shown in FIGS. 1B and 1C. According to some implementations, as shown in FIG. 1B, the injector 13 first translates from the ready position to the injection position prior to the plunger 32 being advanced or appreciably advanced in the reservoir 14. The method of embedding a segment of the distal end portion 16a in the septum 17 while the injector 13 is in the ready position is one way of facilitating such an action. That is, as a result of the distal opening of the needle 16 being closed off while the injector 13 is in the ready position, a hydraulic lock is created in the reservoir 14 that inhibits a movement of the plunger 32 in the reservoir as the spring element initially acts on the plunger shaft 15.

Upon the injector 13 assuming the injection position with the distal end portion 16a of the needle 16 residing outside the housing 11, the plunger 32 is advanced through the reservoir 14 as a result of the spring element 18 continuing to act on the plunger shaft 18. Such action results in the hypoglycemia treatment agent being expelled through the needle 16 and into the patient. According to some implementations the injector 13 contains a single dose of the hypoglycemia treatment agent and is configured to expel the entirety of the single dose upon being activated. It is appreciated that the actuator 21 and stop 24 assembly may take many forms and that the construction of the assembly is not limited to the examples provided herein. According to some implementations the actuator 21 is configured to cause the stop 24 to pivot from the first position to the second position. According to other implementations the actuator 21 is configured to cause the stop 24 to vertically retract into the housing of the actuator 21. According to other implementations the actuator 21 and stop 24 assembly may comprise an electrically activated shutter assembly that functions similar to shutter assemblies found in some cameras. In such an example when the shutter is closed the spring element 18 is prevented from acting on the plunger shaft 15, and when the shutter is open the spring element 18 is able to act on the plunger shaft 15.

According to some implementations the actuator 21 is configured to magnetically act on the stop 24 to cause the stop to move between the first and second positions. According to such implementations the housings 11 and 12 of the assembly 10 need not possess an opening to accommodate a mechanical coupling of the actuator 21 with the stop 24. According to some implementations at least a portion of the stop 24 comprises a metal that is capable of being attracted or repelled by a magnetic field generated by the actuator 21 upon the actuator being energized. According to some implementations at least a portion of the stop 24 comprises a ferromagnetic material. According to some implementations the stop 24 moves between the first position to the second position by rotational or translational movement when exposed to the magnetic forces generated by the actuator 21.

According to some implementations the controller 20 delays activating the actuator 21 for a predetermined amount of time after having initiated the alarm 22 or after having received a signal from the glucose sensor A indicative of hypoglycemia being detected in the patient. The delay is provided so that upon the patient being awakened he/she may abort the impending injection by acting on a manually activated abort switch 23 connected with the controller 20. The switch 23 may be located on a top or side surface of the device 10. In order to prevent inadvertent activation of the abort switch 23, the abort switch 23 may have a cover (not shown in the figures) that must be removed in order to access the switch. According to some implementations when the abort switch 23 comprises a push button, the button may first require that it be twisted before it can be depressed. According to implementations when the abort switch 23 is touch activated, a cover that must be peeled back to expose the switch may be provided to prevent inadvertent activation. In any event, it is desirable that the protective features enabling the activation of the abort switch 23 be sufficiently complex to hinder inadvertent activation by the patient while awake or asleep.

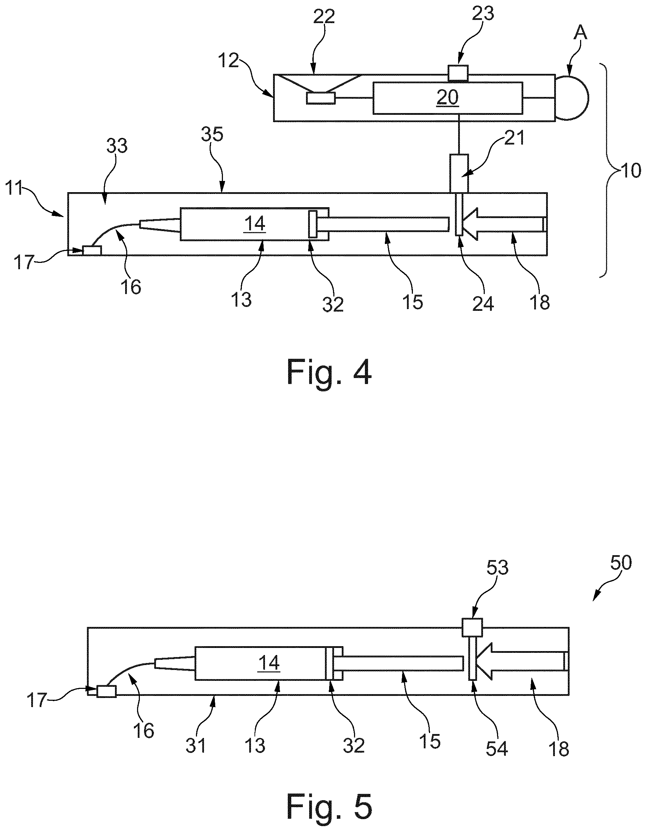

As illustrated in FIGS. 1A-1C and FIG. 4, according to some implementations the device 10 comprises a first housing 11 and a second housing 12. According to some implementations, but not all, the inner chamber 33 of the first housing 11 is maintained in a sterile condition at least until the injector 13 has been activated and the distal end portion 16a of the needle 16 completely penetrates the septum 17. The inner chamber of the second housing 12 that includes a majority, if not all, of the electrical components 20, 21, 22 and 23 need not be maintained in a sterile condition. For this reason, according to some implementations the inner chamber 33 of the first housing 11 is maintained in a sterile condition while the inner chamber of the second housing 12 is not. The use of first and second housings 11 and 12 provides several advantages. First it permits a modular construction in which a first set of components that require a sterile environment reside in the first housing 11 and a second set of components that do not require a sterile environment reside in the second housing 12. The modular construction allows that components residing in the inner chamber 33 of the first housing 11 be sterilized prior to there being an interconnection with the second housing 12. In the present example this reduces sterilization costs by isolating the sterilization area to the chamber 33 that houses the injector 13. It also advantageously obviates the need to sterilize the electronic components that are typically susceptible to temperature and/or chemical degradation when presented in a sterilization inducing environment. Further, in some circumstances the shelf-life of one module may exceed the shelf-life of the other module. In such circumstances a modular design allows that the module having the shorter shelf-life be periodically replaced while keeping the module having the longer shelf-life in place. For example, in the implementation of FIGS. 1A-C the treatment agent residing in reservoir 14 may have a shelf-life of days or weeks after the device 10 has been applied to the patient, while the shelf-life of the components in the second housing 12 may be much longer. By equipping the emergency injection device 10 with quick disconnect features that enables the first housing 11 and its associated components to be disconnected from the second housing 12 and its associated components, the patient is able to periodically replace the first housing 11 without having to replace the second housing 12 resulting in a cost savings.

According to other implementations the inner chamber 33 of the housing 11 is not maintained in a sterile condition when the injector 13 is in the ready position. In such implementations the reservoir 14 of the injector 13 may by itself provide a sterile containment for the treatment agent. Further, prior to use the needle 16 may be contained in a sterile sleeve that collapses upon the needle 16 being advanced within the housing 11 during an injection event. In such implementations the septum 17 is not required and the distal end of the needle 16 is capable of piercing through a distal end of the sleeve as the injector 13 and needle 16 are advanced in the housing 11. Further, according to some implementations the injector 13 is removable so that when the shelf-life of the treatment agent in the reservoir 14 is about to be exceeded the injector 13, along with the needle 16, may be replaced.

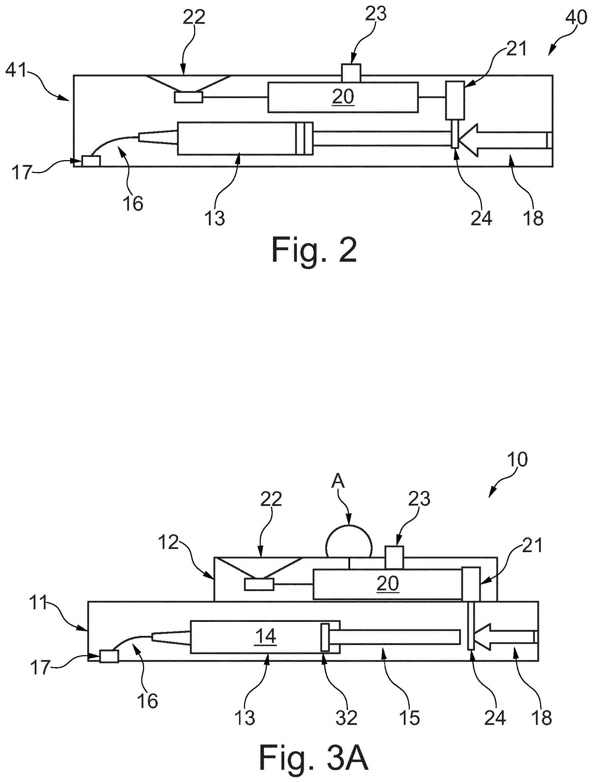

In the implementation of FIGS. 1A-C the first and second housings 11 and 12 are physically attached to one another in the post-manufactured state. In the implementation of FIG. 4 the housings 11 and 12 are physically separated from one another in the post-manufactured state. In either of the implementations of FIGS. 1A-C or FIG. 4, a seal may be maintain at the upper wall 35 of the housing 11 at a location where either the actuator 21 or stop 24 breaches the housing 11. As previously discussed, the actuator 21 and stop 24 may take many forms, and in some instances may commonly reside inside the first housing 11 (as shown in FIG. 2) with only an electrical connector passing through the upper wall 35. In such a case, the seal in the upper wall 35 would reside about the electrical connector. Notwithstanding the foregoing, as shown in FIG. 2, all of the components of the emergency injection device 10 may reside in a single housing 41.

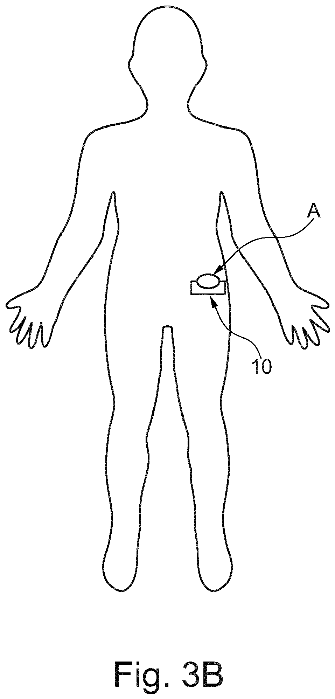

As explained above, FIG. ID illustrates a system according to one implementation that includes an emergency injection device 10 and a separate stand-alone glucose sensor A that may be worn spaced-apart from device 10. As previously described, the glucose sensor A may have a short-range radio-emitting device that emits signals indicative of the blood glucose level of the patient and the emergency injection device 10 may have a receiver for receiving the signals emitted by the glucose sensor A. According to other implementations the glucose sensor A is integrated into or otherwise structurally coupled with the emergency injection device 10 as shown in FIGS. 3A-B and FIG. 4. In such implementations the glucose sensor A may communicate with the controller 20 via a wired connection as shown in FIG. 3A and FIG. 4. It is important to appreciate however, that according to any of the aforementioned implementations the glucose sensor A may communicate with the controller 20 via wired or wireless communications.

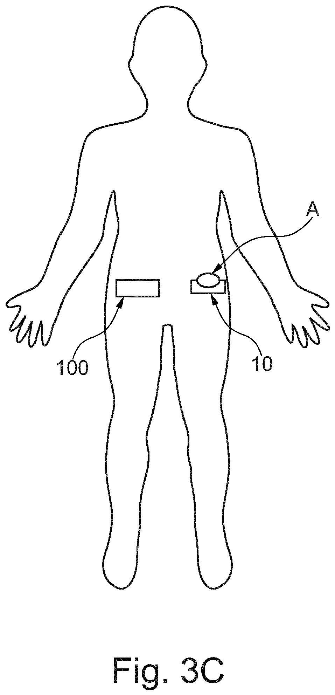

FIG. IE and FIG. 3C each illustrate a system comprising an emergency injection device 10, an insulin pump 100 and a glucose sensor A. In the system of FIG. IE the glucose sensor A is a stand-alone component that may be worn spaced-apart from the emergency injection device 10 and the insulin pump 100 injection site. In such an implementation the glucose sensor A may include a short-range signal generator that is capable of communicating with the signal receiver of the emergency injection device 10 and also a signal receiver of the insulin pump 100. A controller associated with each of devices 10 and 100 processes the signals received from the glucose sensor A with the processor of the insulin pump 100 regulating the infusion of insulin to the patient.

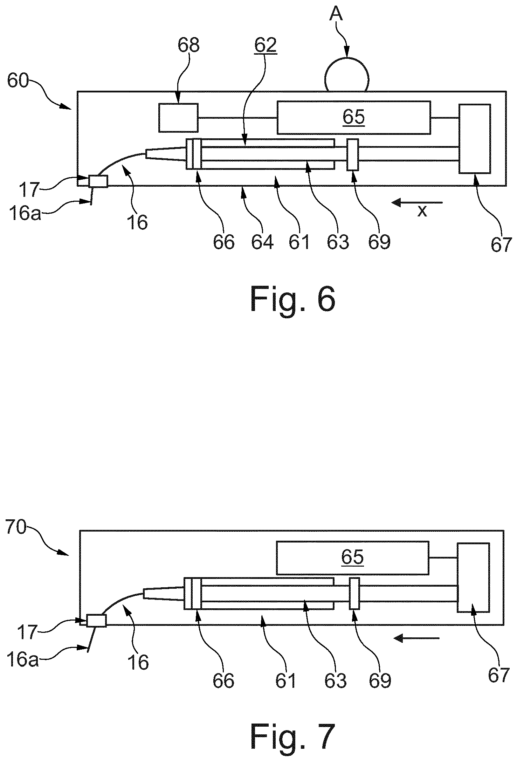

According to other implementations, as noted above, the glucose sensor A may be integrated into or otherwise structurally coupled to the emergency injection device 10. Thus, in a system according to the implementation of FIG. 3C the glucose sensor A may communicate with the emergency injection device 10 via a wired connection and with the insulin pump 100 via a wireless connection. In situations when the glucose sensor A forms a part of the emergency injection device 10, the device 10 may include a signal transmitter that forms a part of or is otherwise coupled to the controller 20. According to such an implementation the glucose sensor A need not comprise a signal generator/transmitter. FIG. 6 illustrates such a configuration and will be discussed in more detail below.

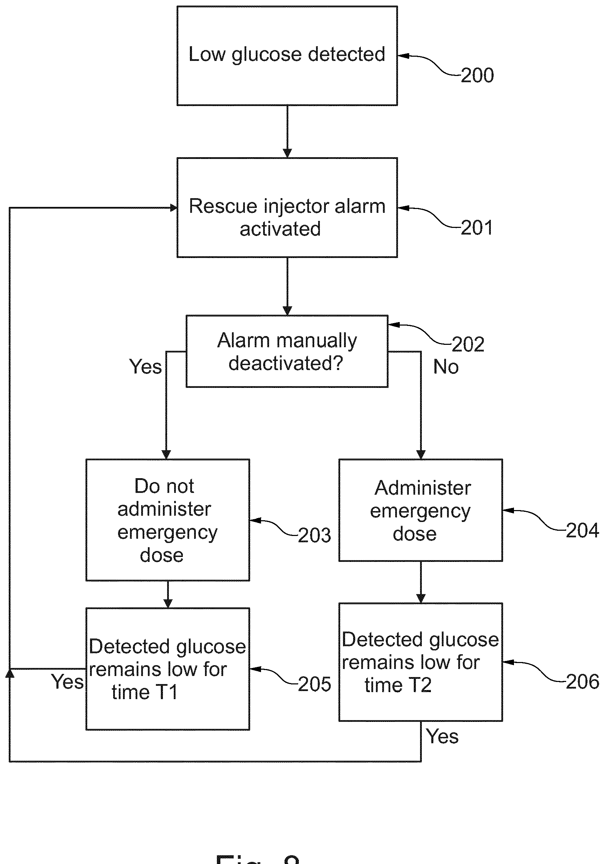

FIG. 8 illustrates a control method of the emergency injection device 10 according to one implementation. Starting at block 200 a low glucose level is detected by the controller 20 as a result of receiving a signal directly or indirectly from a glucose sensor A. At block 201 the alarm 22 is activated to alert the patient of the low glucose level. At block 202 it is determined whether the patient has manually deactivated the alarm 22 by acting on the manually activated abort switch 23. If the alarm 22 has not been manually deactivated by the patient after a predetermined amount of time, at block 204 the device 10 administers by use of the injector 13 the emergency dose of the treatment agent to the patient. According to some implementations, after having had administered the emergency dose to the patient the glucose level of the patient continues to be monitored, and if at block 206 it is detected that the glucose level remains low for a time T2 after the emergency dose has been administered, the alarm 22 is again activated to alert the patient at block 201. If at block 202 it is determined that the patient has acted upon the abort switch 23 within the predetermined amount of time, administration of the emergency dose is aborted at block 203. According to some implementations the glucose level of the patient continues to be monitored and if the detected glucose level remains low for a time T1 after the alarm 22 has been deactivated by the patient, the alarm 22 is again activated at block 201 to alert the patient of the detected low blood glucose level. According to some implementations, in such an instance the protocol according to blocks 202 through 206 may again be implemented.

According to some implementations one or both of blocks 205 and 206 need not be a part of the control method. However, in circumstances in which control blocks 205 and 206 constitute a part of the control method, according to some implementations time T1 is equal to or less than time T2. According to some implementations times T1 and T2 may individually vary between 10 to 45 minutes, and preferably between 15 to 30 minutes.

In accordance with the foregoing the controller 20 of the emergency injection device 10 may be configured to perform the method of: (i) processing a received signal to determine that a low glucose level is detected, (ii) activating the alarm 22 to alert the patient when a low glucose level is detected, (iii) after a predetermined amount of time of activating the alarm 22, administering an emergency dose of the hypoglycemia treatment agent to the patient, (iv) at a time T after the emergency dose has been administered to the patient, receiving a signal to determine if a low glucose level persists, and (v) upon determining that a low glucose level persists, activating the alarm 22 again to alert the patient.

Further, in accordance with the foregoing, the controller 20 of the emergency injection device 10 may be configured to perform the method of, (i) processing a received signal to determine that a low glucose level is detected, (ii) activating the alarm 22 to alert the patient when a low glucose level is detected, (iii) delaying the administration of an emergency dose of a hypoglycemia treatment agent for a predetermined amount of time after activating the alarm 22, (iv) determining during the predetermined amount of time whether the patient has manually deactivated the alarm 22 via the manually activated switch 23, and (v) upon determining that the alarm 22 has been deactivate aborting the administration of the emergency dose. According to some implementations the controller 20 is further configured to carry out the method of activating the alarm 22 again if the low glucose level persists a predetermined amount of time after the user deactivates the alarm.

FIGS 6 and 7 show alternative types of injection devices 60 and 70, respectively, wherein a motor 67 under the control of a controller 65 acts on a drive shaft 63 to advance a plunger 66 through a reservoir 62 of an injector 61. Devices 60 and 70 may be used for emergency purposes as discussed above, or for emulating the function of a pancreas by periodically injecting hypoglycemia treatment agent such as glucagon into the patient on an as-needed basis. According to some implementations the motor 67 has or is coupled to a part 69 that is advanced in the direction of arrow X when the motor 67 is activated by the controller 65. As with some of the implementations described above, the chamber that houses the injector 61 may be maintained in a sterile state with a septum 17 located along the bottom wall 64 of the housing providing a sterile barrier to the outside of the injection device.

According to some implementations the injector 61 is able to translate in the direction of arrow X from an initial ready position to an injection position. As described above, in such an implementation the advancement of the injector 61 causes the distal end portion 16a of the needle 16 to be advanced through the septum 17 and into the patient. FIGS 6 and 7 show the needle 16 after being advanced through the septum 17.

The injector 61 includes a reservoir 62 containing an agent for treating hypoglycemia, such as glucagon. The needle 16 possesses an internal through lumen that extends between proximal and distal ends of the needle with the proximal end of the needle 16 being in fluid communication with the reservoir 62. The plunger 66 located within the reservoir 62 is moveable from a first position typically located near a proximal end of the reservoir to a second position typically located near a distal end of the reservoir and functions to expel the treatment agent from the reservoir 62 upon the shaft 63 coupled to the plunger 33 being acted upon by part 69 that is coupled to the motor 67. According to some implementations the injector 61 is removable so that when the shelf-life of the treatment agent in the reservoir 62 is about to be exceeded the injector 61, along with the needle 16, may be replaced.

According to some implementations, the inner chamber that houses the injector 61 is maintained in a sterile condition at least when the injector 61 is in the ready position. As noted above, a septum 17 located in the bottom wall 64 of the housing provides an exit port by which the distal end portion 16a of the needle 16 may be advanced to a position outside the device housing. The construction and material of each of the needle 16 and the septum 17 enables the distal end portion 16a of the needle 16 to be advanced through the septum 17 as the injector is moved from the ready position to the injection position. According to one implementation the needle 16 is made of a flexible or semi-flexible metal or plastic material. The needle 16 may comprise any type of conduit capable of transporting the hypoglycemia treatment agent from the reservoir 62 to the injection site of the patient. According to some implementations, when the injector 61 is in the ready position at least a segment of the distal end portion 16a of the needle 16 is positioned inside the septum 17, being embedded in the septum 17 as shown in FIG. 9A or otherwise residing in a recess the septum 17 as shown in FIG. 9B. According to some implementations at least a segment of the distal end portion 16a of the needle 16 is embedded in the septum 17 as a result of, for example, having been pierced through a portion of the septum 17. According to some implementations the segment residing in the septum 17 when the injector 61 is in the ready position is oriented perpendicular, or substantially perpendicular, to the bottom wall 64 of the housing. When the needle 16 is advanced through the septum 17, such an arrangement encourages the distal end of the needle 16 to take a path in alignment with the perpendicular orientation. That is, it encourages the distal end portion 16a to assume a predominately perpendicular orientation with respect to the bottom wall 64 when the distal end portion is advanced to reside outside the housing.

Each of devices 60 and 70 includes a controller 65 that directly or indirectly communicates with a glucose sensor A. In the implementation of FIG. 6, the glucose sensor A is integrated with or otherwise structurally coupled to the device 60 and communicates with the controller 65 via a wired connection. In the implementation of FIG. 7 a glucose sensor A does not form a part of the injection device 70. In situations where it is desired that the injection device 70 be at least partially controlled by the output of a glucose sensor A, the injection device 60 is equipped with a signal receiver capable of receiving a wireless signal from the glucose sensor A. The signal receiver may form a part of the controller 65 or be a separate component that communicates with the controller 65. With respect to each of injection devices 60 and 70, the controller 65 is configured to process signals received from the glucose sensor A and to periodically administer doses of the treatment agent upon specific glucose levels being detected. The controller 65 may be electrically coupled to an alarm (not shown), and when so coupled may be configured to activate the alarm when it is determined that hypoglycemia is detected in the patient. According to some implementations the alarm may be an acoustical alarm that produces a sound sufficient to wake the patient when the patient is asleep. According to some implementations the acoustical alarm produces sounds of greater than 50 decibels, 60 decibels, 70 decibels, 80 decibels or 90 decibels. In lieu of the acoustical alarm or in conjunction therewith, the alarm may also comprise a vibrating element, electrical stimulator or other element or set of elements constructed to stimulate a patient's sense of touch.

The injection device 60 may form a part of a system similar to that depicted in FIG. 3C. That is, it may function in conjunction with an insulin pump 100 to properly control the blood glucose level of a patient. According to the implementation of FIG. 6 where the glucose sensor A forms a part of the injection device 60, the injection device 60 is provide with a signal transmitter 68 that is directly wired to the glucose sensor A or indirectly wired to the glucose sensor A via controller 65. In any event, the signal transmitter 68 emits signals that are capable of being received and processed by the insulin pump 100.

The injection device 70 may form a part of a system similar to that depicted in FIG. IE. That is, it may function in conjunction with a separate glucose sensor A and an insulin pump 100 to properly control the blood glucose level of a patient. According to such an implementation, and according to the same principles of the system of FIG. IE, the glucose sensor A may wirelessly communicate with each of the injection device 70 and the insulin pump 100 to control a patient's blood glucose levels.

FIG. 5 illustrates a purely mechanical activated emergency injection device 50 according to one implementation. The device 50 includes an injector 13 similar to that disclosed in conjunction with the implementation of FIGS. 1A-C. The injector 13 includes a reservoir 14 containing a hypoglycemia treatment agent such as glucagon. The treatment agent is administered to the patient via a needle 16, that when deployed has a distal end that protrudes through a septum 17 located along the bottom wall 31 of the housing 11. The treatment agent is expelled from the reservoir 14 into a proximal end of the needle 16 upon the spring element 18 acting on the plunger shaft 15 to move the plunger 32 in the direction of arrow X. The plunger 32 located within the reservoir 14 is moveable from a first position typically located near a proximal end of the reservoir to a second position typically located near a distal end of the reservoir and functions to expel the treatment agent from the reservoir 14 as it is advanced distally through the reservoir.

According to some implementations the injector 13 is able to translate in the direction of arrow X from an initial ready position to an injection position upon the spring element 18 initially acting on the plunger shaft 15. As described above, in such an implementation the advancement of the injector 13 causes the distal end 16a of the needle to be advanced through the septum 17 and into the patient. FIG. 5 shows the needle 16 before being advanced through the septum 17.

With continued reference to FIG. 5, according to some implementations, the inner chamber 33 that houses the injector 13 is maintained in a sterile condition at least when the injector 13 is in the ready position. As noted above, a septum 17 located along the bottom wall 31 of the housing 11 provides an exit port by which the distal end portion 16a of the needle 16 may be advanced to a position outside the device housing. The construction and material of each of the needle 16 and the septum 17 enables the distal end portion 16a of the needle 16 to be advanced through the septum 17 as the injector is moved from the ready position to the injection position. According to one implementation the needle 16 is made of a flexible or semi-flexible metal or plastic material. The needle 16 may comprise any type of conduit capable of transporting the hypoglycemia treatment agent from the reservoir 62 to the injection site of the patient. According to some implementations, when the injector 61 is in the ready position at least a segment of the distal end portion 16a of the needle 16 is positioned inside the septum 17, being embedded inside the septum as shown in FIG. 9A or otherwise residing in a recess of the septum 17 as shown in FIG. 9B. According to some implementations at least a segment of the distal end of the needle 16 is embedded in the septum 17 as a result of, for example, having been pierced through a portion of the septum 17. According to some implementations the segment residing in the septum 17 when the injector 61 is in the ready position is oriented perpendicular, or substantially perpendicular, to the bottom wall 31 of the housing. When the needle 16 is advanced through the septum 17, such an arrangement encourages the distal end of the needle 16 to take a path in alignment with the perpendicular orientation. That is, it encourages the distal end of the needle 16 to assume a predominately perpendicular orientation with respect to the bottom wall 31 when the distal end portion is advanced to reside outside the housing.

Further, although the implementations described have been discloses as comprising computer apparatus and processes performed in computer apparatus, the invention also extends to computer programs, particularly computer programs on or in a carrier, adapted for putting the invention into practice. The program may be in the form of source code, object code, a code intermediate source and object code such as in partially compiled form, or in any other form suitable for use in the implementation of the processes according to the invention. The carrier may be any entity or device capable of carrying the program. For example, the carrier may comprise a storage medium, such as a ROM, for example a CD ROM or a semiconductor ROM, or a magnetic recording medium. Further, the carrier may be a transmissible carrier such as an electrical or optical signal which may be conveyed via electrical or optical cable or by radio or other means. When the program is embodied in a signal which may be conveyed directly by a cable or other device or means, the carrier may be constituted by such cable or other device or means. Alternatively, the carrier may be an integrated circuit in which the program is embedded, the integrated circuit being adapted for performing, or for use in the performance of, the relevant processes.

While the above description contains many specifics, those specifics should not be construed as limitations on the scope of the disclosure, but as non-limiting exemplifications of preferred implementations thereof.

## Claims

1. An injection device adapted to be worn on the body of a patient, the device comprising:
an injector comprising a reservoir containing an agent for treating hypoglycemia and a needle comprising a first end portion connected to the reservoir and a second end portion adapted to be inserted into the body of the patient;
an acoustical alarm; and
a control assembly comprising a signal receiver configured to receive signals from a glucose sensor, the control assembly being electrically coupled to the acoustical alarm and configured to activate the acoustical alarm upon a signal received in the signal receiver being indicative of hypoglycaemia, the control assembly being configured to automatically cause the injector to administer through the needle a dose of the agent in the reservoir to the patient after a predetermined period of time from the signal receiver receiving the signal indicative of hypoglycaemia, the control assembly having a manually activated switch that when activated prior to the expiration of the predetermined period of time causes the control assembly to abort the automatic injection of the agent.

2. An injection device according to claim 1, wherein the injector resides in a sterile chamber inside a first housing, the first housing having a bottom wall with a through opening that extends between an inner surface and an outer surface, a portion of the outer surface that possesses the through opening is configured to be worn in contact with the body of the patient, a septum positioned in and/or over the through hole opening that assists in maintaining the sterile integrity of the sterile chamber, the second end portion of the needle being movable between a first position where the second end portion of the needle partially resides in the septum and a second position where the second end portion of the needle extends through the septum and at least partially resides external to the bottom wall, the septum and second end portion of the needle being constructed so that the second end portion of the needle is capable of penetrating the septum when the second end portion of the needle is moved between the first position and the second position.

3. An injection device according to claim 1 or claim 2 wherein the reservoir contains a single dose of the agent for treating hypoglycaemia.

4. An injection device according to any one of the preceding claims, wherein the injector comprises a syringe assembly that includes the reservoir, the needle extending distally to the reservoir, a plunger residing in the reservoir and connected to a shaft that extends proximally from the reservoir, the control assembly including an electrical actuator that controls the positioning of a stop between a first position and a second position, in the first position the stop resides between a spring element and the plunger shaft to prevent the spring element from acting on the plunger shaft, and in the second position the stop does not reside between the spring element and the plunger to permit the spring element to act on the plunger shaft to move the plunger distally within the reservoir to cause the agent in the reservoir to be dispensed through the needle.

5. An injection device according to claim 4, wherein the syringe assembly is configured to shift a distance distally within the sterile chamber upon the spring element initially contacting the plunger shaft to enable the second end portion of the needle to move from the first position to the second position.

6. An injection device according to claim 5, wherein the syringe assembly is configured to shift the distance distally within the sterile chamber prior to the plunger being moved within the reservoir.

7. An injection device according to claim 1 or claim 2, wherein the injector comprises a syringe assembly that includes the reservoir, the needle extending distally to the reservoir, a plunger residing in the reservoir and connected to a shaft that extends proximally from the reservoir, the control assembly including an electric motor assembly coupled to the plunger shaft and configured to act on the plunger shaft to move the plunger distally within the reservoir to cause the agent in the reservoir to be dispensed through the needle.

8. An injection device according to claim 7, wherein the syringe assembly is configured to shift a distance distally within the sterile chamber upon the electric motor assembly initially acting on the plunger shaft to enable the second end portion of the needle to move from the first position to the second position.

9. An injection device according to claim 8, wherein the syringe assembly is configured to shift the distance distally within the sterile chamber prior to the plunger being moved within the reservoir.

10. An injection device according to any one of the preceding claims wherein the signal receiver is configured to receive wireless signals from the glucose sensor.

11. An injection device according to any one of claims 1 to 9, wherein the glucose sensor is physically coupled to the device and is electrically coupled to the signal receiver.

12. An injection device according to claim 2, wherein the injector, acoustical alarm and control assembly reside inside the sterile chamber.

13. An injection device according to claim 2, wherein the injector resides in the sterile chamber within the first housing, and the signal receiver and acoustical alarm reside in a second housing.

14. An injection device according to claim 13, wherein the first and second housings are spaced apart from one another.

15. An injection device according to any one of the preceding claims wherein, following the first abort, the control assembly is further configured to:
(a) reactivate the acoustical alarm upon the signal receiver receiving a continued signal indicative of hypoglycemia after a second predetermined period of time; and
(b) following the second predetermined period of time, to automatically cause the injector to administer through the needle the dose of the agent in the reservoir to the patient unless the manually activated switch is further activated prior to the expiration of a third predetermined period of time.

## Patentansprüche

1. Injektionsvorrichtung, die angepasst ist, um am Körper eines Patienten getragen zu werden, wobei die Vorrichtung Folgendes umfasst:
einen Injektor, der einen Vorratsbehälter, der ein Mittel zur Behandlung von Hypoglykämie enthält, und eine Nadel umfasst, die einen ersten Endabschnitt, der mit dem Vorratsbehälter verbunden ist, und einen zweiten Endabschnitt umfasst, der angepasst ist, um in den Körper des Patienten eingeführt zu werden;
einen akustischen Alarm; und
eine Steueranordnung, die einen Signalempfänger umfasst, der konfiguriert ist, um Signale von einem Glucosesensor zu empfangen, wobei die Steueranordnung elektrisch mit dem akustischen Alarm gekoppelt ist und konfiguriert ist, um bei Empfang eines Signals im Signalempfänger, das eine Hypoglykämie anzeigt, einen akustischen Alarm zu aktivieren, wobei die Steueranordnung konfiguriert ist, den Injektor automatisch zu veranlassen, nach einer vorbestimmten Zeitdauer nach Empfang des Signals, das eine Hypoglykämie anzeigt, vom Signalempfänger, durch die Nadel eine Dosis des Mittels im Vorratsbehälter an den Patienten zu verabreichen, wobei die Steueranordnung einen manuell aktivierten Schalter aufweist, der bei Aktivierung vor Ablauf der vorbestimmten Zeitdauer bewirkt, dass die Steueranordnung die automatische Injektion des Mittels abbricht.

2. Injektionsvorrichtung nach Anspruch 1, wobei der Injektor in einer sterilen Kammer innerhalb eines ersten Gehäuses enthalten ist, wobei das erste Gehäuse eine Bodenwand mit einer Durchgangsöffnung umfasst, die sich zwischen einer Innenoberfläche und einer Außenoberfläche erstreckt, wobei ein Teil der Außenwand, der die Durchgangsöffnung aufweist, konfiguriert ist, um in Kontakt mit dem Körper des Patienten getragen zu werden, ein Septum in und/oder über der Durchgangsöffnung positioniert ist und dazu beiträgt, die sterile Unversehrtheit der sterilen Kammer aufrechtzuerhalten, wobei der zweite Endabschnitt der Nadel zwischen einer ersten Position, in welcher der zweite Endabschnitt der Nadel teilweise in dem Septum angeordnet ist, und einer zweiten Position, in welcher der zweite Endabschnitt der Nadel sich durch das Septum erstreckt und zumindest teilweise außerhalb der Bodenwand ist, bewegbar ist, wobei das Septum und der zweite Endabschnitt der Nadel so konstruiert sind, dass der zweite Endabschnitt der Nadel in der Lage ist, das Septum zu durchdringen, wenn der zweite Endabschnitt der Nadel zwischen der ersten Position und der zweiten Position bewegt wird.

3. Injektionsvorrichtung nach Anspruch 1 oder Anspruch 2, wobei der Vorratsbehälter eine einzelne Dosis des Mittels zur Behandlung von Hypoglykämie enthält.

4. Injektionsvorrichtung nach einem der vorangegangenen Ansprüche, wobei der Injektor eine Spritzenanordnung, die den Vorratsbehälter umfasst, wobei sich die Nadel distal zum Vorratsbehälter erstreckt, und einen Kolben, der im Vorratsbehälter angeordnet ist und mit einem Schaft verbunden ist, der sich proximal vom Vorratsbehälter erstreckt, umfasst, wobei die Steueranordnung einen elektrischen Aktuator umfasst, der die Positionierung eines Stopps zwischen einer ersten Position und einer zweiten Position steuert, wobei in der ersten Position der Stopp zwischen einem Federelement und dem Kolbenschaft angeordnet ist, um zu verhindern, dass das Federelement auf den Kolbenschaft einwirkt, und in der zweiten Position der Stopp nicht zwischen dem Federelement und dem Kolben angeordnet ist, um zu ermöglichen, dass das Federelement auf den Kolbenschaft einwirkt, um den Kolben distal innerhalb des Vorratsbehälters zu bewegen und zu bewirken, dass das Mittel im Vorratsbehälter durch die Nadel ausgegeben wird.

5. Injektionsvorrichtung nach Anspruch 4, wobei die Spritzenanordnung konfiguriert ist, um sich einen Abstand distal innerhalb der sterilen Kammer zu verschieben, wenn das Federelement beginnt, mit dem Kolbenschaft in Kontakt zu treten, um es dem zweiten Endabschnitt der Nadel zu ermöglichen, sich von der ersten Position in die zweite Position zu bewegen.

6. Injektionsvorrichtung nach Anspruch 5, wobei die Spritzenanordnung konfiguriert ist, um sich den Abstand distal innerhalb der sterilen Kammer zu verschieben, bevor der Kolben innerhalb des Vorratsbehälters bewegt wird.

7. Injektionsvorrichtung nach Anspruch 1 oder 2, wobei der Injektor eine Spritzenanordnung umfasst, die den Vorratsbehälter umfasst, wobei sich die Nadel distal zum Vorratsbehälter erstreckt, ein Kolben in dem Vorratsbehälter angeordnet ist und mit einem Schaft verbunden ist, der sich proximal vom Vorratsbehälter erstreckt, wobei die Steueranordnung eine Elektromotoranordnung umfasst, die mit dem Kolbenschaft gekoppelt ist und konfiguriert ist, um auf den Kolbenschaft zu wirken, um den Kolben distal innerhalb des Vorratsbehälters zu bewegen, um zu bewirken, dass das Mittel im Vorratsbehälter durch die Nadel abgegeben wird.

8. Injektionsvorrichtung nach Anspruch 7, wobei die Spritzenanordnung konfiguriert ist, um sich einen Abstand distal innerhalb der sterilen Kammer zu verschieben, wenn die Elektromotoranordnung beginnt, auf den Kolbenschaft zu wirken, um es dem zweiten Endabschnitt der Nadel zu ermöglichen, sich von der ersten Position in die zweite Position zu bewegen.

9. Injektionsvorrichtung nach Anspruch 8, wobei die Spritzenanordnung konfiguriert ist, um sich den Abstand distal innerhalb der sterilen Kammer zu verschieben, bevor der Kolben im Vorratsbehälter bewegt wird.

10. Injektionsvorrichtung nach einem der vorangegangenen Ansprüche, wobei der Signalempfänger konfiguriert ist, um drahtlos Signale vom Glucosesensor zu empfangen.

11. Injektionsvorrichtung nach einem der Ansprüche 1 bis 9, wobei der Glucosesensor physisch mit der Vorrichtung gekoppelt und mit dem Signalempfänger elektrisch gekoppelt ist.

12. Injektionsvorrichtung nach Anspruch 2, wobei der Injektor, der akustische Alarm und die Steueranordnung innerhalb der sterilen Kammer angeordnet sind.

13. Injektionsvorrichtung nach Anspruch 2, wobei der Injektor in der sterilen Kammer innerhalb des ersten Gehäuses angeordnet ist und der Signalempfänger und der akustische Alarm in einem zweiten Gehäuse angeordnet sind.

14. Injektionsvorrichtung nach Anspruch 13, wobei das erste und das zweite Gehäuse voneinander beabstandet sind.

15. Injektionsvorrichtung nach einem der vorangegangenen Ansprüche, wobei die Steuervorrichtung konfiguriert ist, um nach dem ersten Abbruch:
(a) den akustischen Alarm zu reaktivieren, wenn der Signalempfänger nach einer zweiten vorbestimmten Zeitdauer ein fortgesetztes Signal empfängt, das eine Hypoglykämie anzeigt; und
(b) nach der zweiten vorbestimmten Zeitdauer automatisch den Injektor zu veranlassen, die Dosis des Mittels im Vorratsbehälter durch die Nadel an den Patienten zu verabreichen, sofern nicht der manuell aktivierte Schalter weiter aktiviert wird, bevor eine dritte vorbestimmte Zeitdauer abgelaufen ist.

## Revendications

1. Dispositif d'injection adapté pour être porté sur le corps d'un patient, le dispositif comprenant :
un injecteur comprenant un réservoir contenant un agent pour traiter l'hypoglycémie et une aiguille comprenant une première partie d'extrémité reliée au réservoir et une seconde partie d'extrémité adaptée pour être insérée dans le corps du patient ;
une alarme sonore ; et
un ensemble de commande comprenant un récepteur de signaux conçu pour recevoir des signaux d'un capteur de glucose, l'ensemble de commande étant couplé électriquement à l'alarme sonore et conçu pour activer l'alarme sonore lorsqu'un signal reçu dans le récepteur de signaux indique une hypoglycémie, l'ensemble de commande étant conçu pour amener automatiquement l'injecteur à administrer par le biais de l'aiguille une dose de l'agent dans le réservoir au patient après une période de temps prédéterminée à partir de la réception par le récepteur de signaux du signal indiquant une hypoglycémie, l'ensemble de commande ayant un commutateur activé manuellement qui, lorsqu'il est activé avant l'expiration de la période de temps prédéterminée, amène l'ensemble de commande à interrompre l'injection automatique de l'agent.

2. Dispositif d'injection selon la revendication 1, dans lequel l'injecteur se trouve dans une chambre stérile à l'intérieur d'un premier logement, le premier logement ayant une paroi inférieure pourvue d'une ouverture traversante qui s'étend entre une surface interne et une surface externe, une partie de la surface externe qui possède l'ouverture traversante est conçue pour être portée en contact avec le corps du patient, un septum positionné dans et/ou sur l'ouverture de trou traversant qui facilite le maintien de l'intégrité stérile de la chambre stérile, la seconde partie d'extrémité de l'aiguille étant mobile entre une première position où la seconde partie d'extrémité de l'aiguille réside partiellement dans le septum et une seconde position où la seconde partie d'extrémité de l'aiguille s'étend à travers le septum et réside au moins partiellement à l'extérieur de la paroi inférieure, le septum et la seconde partie d'extrémité de l'aiguille étant construits de telle sorte que la seconde partie d'extrémité de l'aiguille est apte à pénétrer dans le septum lorsque la seconde partie d'extrémité de l'aiguille est déplacée entre la première position et la seconde position.

3. Dispositif d'injection selon la revendication 1 ou la revendication 2, dans lequel le réservoir contient une seule dose de l'agent destiné à traiter l'hypoglycémie.

4. Dispositif d'injection selon l'une quelconque des revendications précédentes, dans lequel l'injecteur comprend un ensemble seringue qui inclut le réservoir, l'aiguille s'étendant de manière distale au réservoir, un piston réside dans le réservoir et relié à une tige qui s'étend de manière proximale par rapport au réservoir, l'ensemble de commande incluant un actionneur électrique qui commande le positionnement d'une butée entre une première position et une seconde position, dans la première position, la butée réside entre un élément de ressort et la tige de piston pour empêcher l'élément de ressort d'agir sur la tige de piston, et dans la seconde position, la butée ne réside pas entre l'élément de ressort et le piston pour permettre à l'élément de ressort d'agir sur la tige de piston pour déplacer le piston de manière distale à l'intérieur du réservoir pour amener l'agent dans le réservoir à être distribué par le biais de l'aiguille.

5. Dispositif d'injection selon la revendication 4, dans lequel l'ensemble seringue est conçu pour se décaler d'une distance de manière distale à l'intérieur de la chambre stérile lorsque l'élément de ressort est initialement en contact avec la tige de piston pour permettre à la seconde partie d'extrémité de l'aiguille de se déplacer de la première position à la seconde position.

6. Dispositif d'injection selon la revendication 5, dans lequel l'ensemble seringue est conçu pour se décaler de la distance de manière distale à l'intérieur de la chambre stérile avant que le piston ne soit déplacé à l'intérieur du réservoir.

7. Dispositif d'injection selon la revendication 1 ou la revendication 2, dans lequel l'injecteur comprend un ensemble seringue qui inclut le réservoir, l'aiguille s'étendant de manière distale par rapport au réservoir, un piston résidant dans le réservoir et relié à une tige qui s'étend de manière proximale par rapport au réservoir, l'ensemble de commande incluant un ensemble de moteur électrique couplé à la tige de piston et conçu pour agir sur la tige de piston pour déplacer le piston de manière distale à l'intérieur du réservoir pour amener l'agent dans le réservoir à être distribué par le biais de l'aiguille.

8. Dispositif d'injection selon la revendication 7, dans lequel l'ensemble seringue est conçu pour se décaler d'une distance de manière distale à l'intérieur de la chambre stérile lorsque l'ensemble de moteur électrique agit initialement sur la tige du piston pour permettre à la seconde partie d'extrémité de l'aiguille de se déplacer de la première position à la seconde position.

9. Dispositif d'injection selon la revendication 8, dans lequel l'ensemble seringue est conçu pour se décaler de la distance de manière distale à l'intérieur de la chambre stérile avant que le piston ne soit déplacé à l'intérieur du réservoir.

10. Dispositif d'injection selon l'une quelconque des revendications précédentes, dans lequel le récepteur de signaux est conçu pour recevoir des signaux sans fil du capteur de glucose.

11. Dispositif d'injection selon l'une quelconque des revendications 1 à 9, dans lequel le capteur de glucose est couplé physiquement au dispositif et est couplé électriquement au récepteur de signaux.

12. Dispositif d'injection selon la revendication 2, dans lequel l'injecteur, l'alarme sonore et l'ensemble de commande résident à l'intérieur de la chambre stérile.

13. Dispositif d'injection selon la revendication 2, dans lequel l'injecteur réside dans la chambre stérile à l'intérieur du premier logement, et le récepteur de signaux et l'alarme sonore résident dans un second logement.

14. Dispositif d'injection selon la revendication 13, dans lequel les premier et second logements sont espacés l'un de l'autre.

15. Dispositif d'injection selon l'une quelconque des revendications précédentes, dans lequel, suite à la première interruption, l'ensemble de commande est conçu en outre pour :
(a) réactiver l'alarme sonore lors de la réception par le récepteur de signaux d'un signal continu indiquant une hypoglycémie après une deuxième période de temps prédéterminée ; et
(b) suite à la deuxième période de temps prédéterminée, amener automatiquement l'injecteur à administrer par le biais de l'aiguille la dose de l'agent dans le réservoir au patient à moins que le commutateur activé manuellement ne soit activé ultérieurement avant l'expiration d'une troisième période de temps prédéterminée.
